# EUROPEAN PATENT APPLICATION

(11) **EP 4 328 221 A1**
(43) Date of publication of application: **28.02.2024**
(21) Application number: 22791021.3
(22) Date of filing: 19.04.2022
(51) Int. Cl.: C07D 401/04, C07B 53/00

(54) **PREPARATION METHOD OF L-NICOTINE**

(30) Priority: 21.04.2021 CN 202110432773
(71) Applicant: HUANGGANG ZY BIOTECHNOLOGY CO., LTD., Huanggang, Hubei 438011 (CN); WUHAN RS PHARMACEUTICALS CO., LTD., Hubei 436070 (CN); Wuhan QR Pharmaceuticals Co., Ltd., Wuhan, Hubei 430223 (CN)
(72) Inventor: SHEN, Litao, Hubei 436070 (CN); LIU, Yaoxiu, Hubei 436070 (CN)
(74) Representative: Gille Hrabal Partnerschaftsgesellschaft mbB Patentanwälte
(86) International application number: PCT/CN2022/087601
(87) International publication number: WO 2022/222914

(57) **Abstract**

The present invention provides a preparation method for L-nicotine, the method can obtain L-nicotine having an optical purity of more than 99.9%, which is much higher than that of similar products in the current market. The total yield of synthesis reaches 50-60%. The reaction materials are cheap and easy to obtain. The operation is simple, environmentally friendly, and is suitable for large-scale industrial production.

## Description

The present application claims priority to the prior application with the patent application No. 202110432773.7 and entitled "PREPARATION METHOD OF L-NICOTINE" filed to the China National Intellectual Property Administration on April 21, 2021, which is incorporated herein by reference in its entirety.

### TECHNICAL FIELD

The present disclosure relates to the technical field of chemical synthesis, and in particular to a preparation method for L-nicotine.

### BACKGROUND

Nicotine, with a chemical name of 3-(1-methylpyrrol-2-yl) pyridine, is a naturally occurring liquid alkaloid with strong physiological activity. Nicotine is usually found mainly in natural tobacco, and has a wide range of applications in the fields of agriculture, pharmaceutical intermediates, and electronic cigarettes.

Currently, commercial nicotine is mainly extracted and purified from plants such as tobacco, and natural nicotine is mainly in a levorotatory form. Tobacco leaves contain various alkaloids that are not easy to separate from each other, so the levorotatory nicotine (L-nicotine) prepared by the extraction method has a relatively low purity, generally less than 95%, and contains many other nicotine impurities that are unhealthy for human body systems, many of which are shown to be carcinogenic. Meanwhile, the extraction and purification of nicotine from plants such as tobacco can be influenced by various factors such as starting materials, climate, land resources, and period. Therefore, the oriented synthesis of L-nicotine by a chemical method is a current research hotspot, which can avoid the defects of low product purity and high limitation of starting materials in the traditional extraction process.

A method for preparing nicotine by reaction with trimethylsilyl-protected pyrrolidone using pyridine as a starting material is reported in the literature Journal of Labelled Compounds and Radiopharmaceuticals, 1977, 9(4), 461-469:

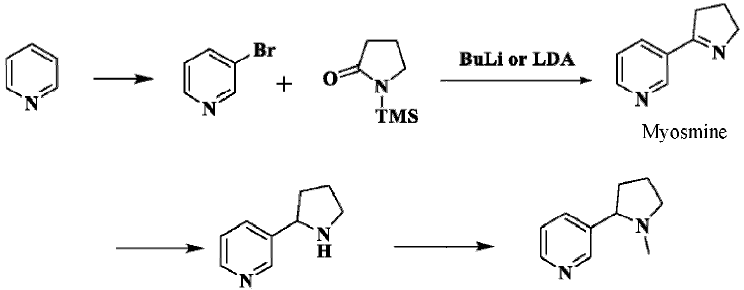

This method requires the use of flammable organic metal lithium and needs to be operated at -78 °C, and the nitrogen atom of the pyrrolidone is protected by trimethylsilane in the route, so the material cost is relatively high.

A method for preparing racemic nicotine by a four-step reaction through a synthetic route using methyl nicotinate as a starting material is disclosed in the literature Organic Syntheses, [J], 1998, 215-218:

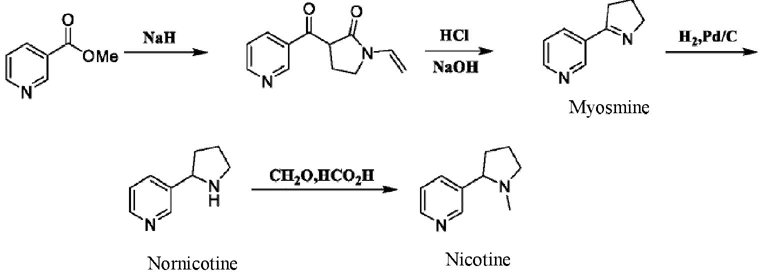

The first two steps of the patented route to synthesize myosmine result in a reaction yield of about 40% and a large amount of tar affecting the purity and appearance of the subsequent finished product.

The patents US2013030188A1 and CN102633773B disclose a method for preparing racemic nicotine by a four-step reaction through a synthetic route using methyl nicotinate and N-butenylpyrrolidone as starting materials:

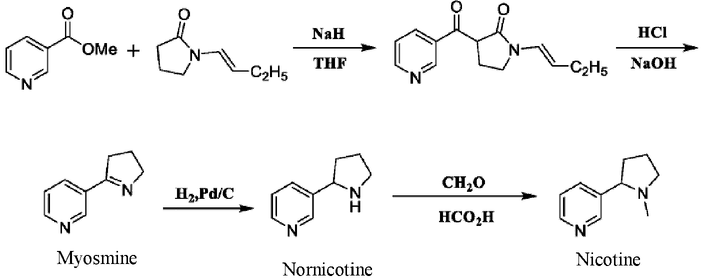

N-butenylpyrrolidone in the route needs to be prepared in-house, which results in a relatively high material cost. Sodium hydride with relatively high risk is used as an alkali, so that a large amount of hydrogen can be generated in the reaction process, and the potential safety hazard is caused in the scale-up production.

The patents EP2484673, US0197022, WO121644, and CN1124093293 disclose a preparation method for L-nicotine by resolution with a cheap chiral acid using racemic nicotine as a starting material.

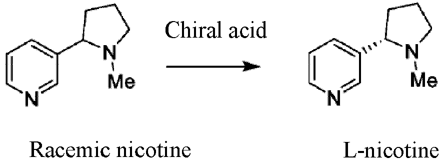

In the route, the racemate is resolved with a cheap chiral acid to obtain L-nicotine, with a single-step resolution yield of about 40%. The resolution process has high cost, with 60% loss of nicotine in the resolution mother liquor; acid and base are required to be poured twice for resolution, so that more three wastes are generated.

The patent CN112409327 discloses a preparation method for target L-nicotine, in which nicotinate is used as a starting material, β-ketonic acid amide is generated under the action of a base, myosmine is obtained by cyclization under an acidic condition, then L-nornicotine is obtained by a biological fermentation method, and finally, the target L-nicotine is obtained by adding methyl.

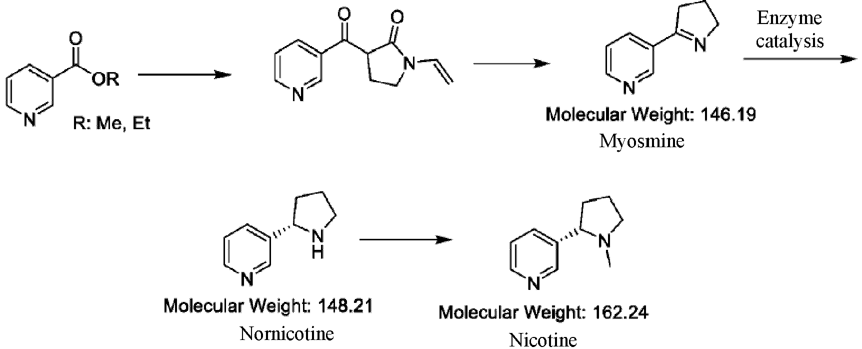

In this method, L-nicotine is obtained by enzyme catalysis, with the cost being 50% lower than that of resolution, but it is a biological product not a fully synthesized product due to the introduction of a trace amount of protein residue in the fermentation process.

Most of nicotine prepared in the prior art is racemic nicotine. In order to obtain homochiral nicotine with high optical purity, a chemical resolution method is required for isolation and purification, which is too complicated; or biological enzyme catalysis is used to obtain L-nicotine, which introduces a trace amount of protein residue, and it is difficult to detect and quantify the residual proteins.

Therefore, in order to meet the demand of the current market for high-purity L-nicotine without pollution of other harmful compounds, there is a need to develop a method for artificially synthesizing L-nicotine, which has relatively high development efficiency and relatively high product purity and is suitable for large-scale industrial production.

### SUMMARY

In order to solve the problems in the prior art, the present disclosure provides a preparation method for L-nicotine having the following synthetic route: 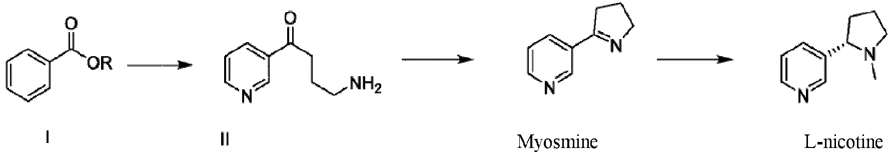 wherein the preparation method comprises the following steps:
(1) subjecting a compound of formula I to a substitution reaction with N-vinylpyrrolidone, and performing decarboxylation to obtain a compound of formula II;
(2) subjecting the compound of formula II to a cyclization reaction to obtain myosmine; and
(3) subjecting myosmine to reduction and methylation to obtain L-nicotine;
in the compound of formula I, R is selected from C₁-C₈ alkyl, phenyl, and benzyl.

According to an embodiment of the present disclosure, the C₁-C₈ alkyl may be selected from methyl, ethyl, *n*-propyl, isopropyl, *n*-butyl, isobutyl, *tert*-butyl, *n*-pentyl, isopentyl, neopentyl, *n*-hexyl, and the like.

According to an embodiment of the present disclosure, in the step (1), the compound of formula I is subjected to the substitution reaction with *N*-vinylpyrrolidone under the action of a base; the base is selected from one or more of potassium hydroxide, sodium hydroxide, potassium *tert*-butoxide, sodium ethoxide, potassium carbonate, sodium hydride, butyl lithium, and methylmagnesium bromide; in the substitution reaction of the step (1), one or more of the following reagents are used as a reaction solvent: toluene, xylene, tetrahydrofuran, ethanol, 2-methyltetrahydrofuran, and *n*-hexane; the substitution reaction is performed at a temperature range of 30-150 °C, preferably 60-120 °C, for example, at a temperature selected from 60 °C, 70 °C, 80 °C, 90 °C, 100 °C, and 110 °C; in the substitution reaction, the reaction solvent and the compound of formula I are at a feeding mass ratio of 20:1-1:1, preferably 10:1-1:1, for example, 8:1, 7:1, 6:1, 6:1, 4:1, 3:1, or 2:1; in the substitution reaction, the base and the compound of formula I are in a feeding mass ratio of 2:1-1:5, for example, 1:1, 1:1.5, 1:2, or 1:2.5; in the substitution reaction, N-vinylpyrrolidone and the compound of formula I are in a feeding mass ratio of 2:1-1:5, for example, 1:1, 1:1.1, 1:1.2, or 1:1.3.

According to an embodiment of the present disclosure, in the step (1), an acid is added for the decarboxylation reaction; the acid is selected from one or more of hydrochloric acid, phosphoric acid, sulfuric acid, formic acid, and acetic acid; preferably, the hydrochloric acid may be selected from hydrochloric acid with a concentration of 15%; the decarboxylation reaction is performed at a temperature range of 10-100 °C, preferably 20-80 °C, for example, at a temperature selected from 30 °C, 40 °C, 50 °C, 60 °C, and 70 °C; preferably, in the decarboxylation reaction, the temperature is controlled in a range of 40-60 °C in the acid addition process (time for the addition may be controlled within 2-4 h), and after the acid is added, the system is heated to 80-100 °C for a reaction (time for the reaction may be controlled within 1-3 h).

According to an embodiment of the present disclosure, the step (1) further comprises the following steps: after the decarboxylation reaction, adjusting pH to 6-8 with a basic reagent, and performing extraction to obtain the compound of formula II; according to an embodiment of the present disclosure, the basic agent may be selected from a sodium hydroxide solution (e.g., 10% sodium hydroxide).

According to an embodiment of the present disclosure, in the step (1), xylene, ethyl nicotinate, potassium *tert*-butoxide, and *N*-vinylpyrrolidone are added to a reaction vessel at 20-40 °C; after the system is heated to 80-120 °C for a reaction for 3-5 h, 15% hydrochloric acid is added dropwise over 2-4 h with the temperature controlled at 30-60 °C; then the system is heated to 80-100 °C for a reaction for 1-3 h, and cooled to room temperature; the system is adjusted to pH 7 with sodium hydroxide and subjected to a post-treatment to obtain the compound of formula II.

According to an embodiment of the present disclosure, in the step (2), the compound of formula II is subjected to the cyclization reaction under the action of a base; the step (2) further comprises the following steps: performing extraction to obtain myosmine.

According to an embodiment of the present disclosure, in the step (2), one or more of the following reagents are used as a reaction solvent: water, ethyl acetate, dichloromethane, *N,N-*dimethylformamide, tetrahydrofuran, ethanol, 2-methyltetrahydrofuran, *n*-hexane, and methyl *tert*-butyl ether.

According to an embodiment of the present disclosure, in the step (2), the base is selected from potassium hydroxide, sodium hydroxide, potassium *tert*-butoxide, sodium ethoxide, potassium carbonate, sodium hydride, triethylamine, butyl lithium, and methylmagnesium bromide.

According to an embodiment of the present disclosure, in the step (2), the reaction is performed at a temperature range of 30-100 °C, for example, at a temperature selected from 40 °C, 50 °C, 60 °C, 70 °C, 80 °C, and 90 °C.

According to an embodiment of the present disclosure, in the step (2), the reaction solvent and the compound of formula II are in a feeding mass ratio of 20:1-2:1, preferably 10:1-3:1, for example, 4:1; the base and the compound of formula II are in a feeding mass ratio of 1:1-1:20, preferably 1:2-1:8, for example, 1:3, 1:4, or 1:5.

According to an embodiment of the present disclosure, in the step (2), the reaction solvent and the compound of formula II are added to a reaction vessel at room temperature, and then sodium hydroxide is added with the temperature of the system controlled at 30-50 °C; after the system is completely reacted at a temperature of 30-50 °C, a post-treatment is performed to obtain myosmine.

According to an embodiment of the present disclosure, in the step (3), the reduction is performed in the presence of a ligand and a metal catalyst; in some embodiments, a chiral catalyst is generated *in situ* from the ligand and the metal catalyst.

According to an embodiment of the present disclosure, in the reduction reaction of the step (3), the reaction solvent and the myosmine are in a feeding mass ratio of 20:1-3:1, preferably 10:1-3:1, for example, 9:1, 8:1, 7:1, 6:1, or 5:1; in the reduction reaction, one or more of the following reagents are used as a reaction solvent: water, 1,4-dioxane, tetrahydrofuran, methanol, glycol dimethyl ether, methyl *tert*-butyl ether, diethyl ether, chloroform, dichloromethane, and the like.

According to an embodiment of the present disclosure, in the step (3), the ligand and the metal catalyst are in a feeding mass ratio of 10:1-1:1, for example, 10:1, 9:1, 8:1, 7:1, 6:1, 5:1, 4:1, 3:1, 2:1, or 1.5:1.

According to an embodiment of the present disclosure, in the step (3), the reduction reaction is hydrogenation reduction, with hydrogen charged into the reaction, and the reaction is performed at a pressure of 0.5-2.0 Mpa, for example, 1.0 Mpa, 1.1 Mpa, 1.2 Mpa, 1.3 Mpa, 1.4 Mpa, or 1.5 Mpa.

According to an embodiment of the present disclosure, in the step (3), the reduction reaction is performed at a temperature of 10-80 °C, for example, at a temperature selected from 20 °C, 30 °C, 40 °C, 50 °C, 60 °C, and 70 °C.

According to an embodiment of the present disclosure, in the step (3), the metal catalyst is selected from Rh(COD)Cl₂, Ir(COD)Cl₂, Ru(COD)Cl₂, PdCl(PPh₃)₃, PdCl₂(PPh₃)₂, Ni(acac)₂, NiCl₂, and Ni(COD)₂.

According to an embodiment of the present disclosure, in the step (3), the ligand is selected from the following structures:

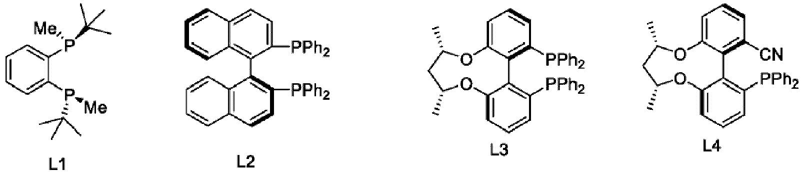

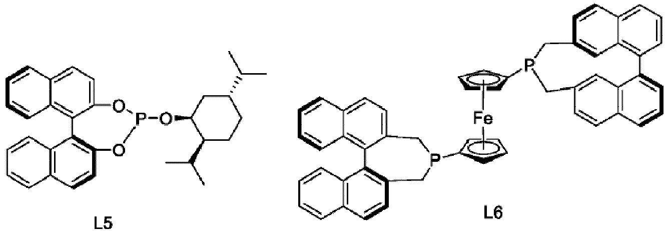

According to an embodiment of the present disclosure, in the step (3), a reagent for the methylation is selected from one or more of formaldehyde (e.g., an aqueous formaldehyde solution), paraformaldehyde, iodomethane, and dimethyl sulfate.

According to an embodiment of the present disclosure, in the methylation reaction of the step (3), the reaction is performed at a temperature range of 40-120 °C, preferably 50-100 °C, for example, at a temperature selected from 60 °C, 70 °C, 80 °C, 90 °C, and 95 °C.

According to an embodiment of the present disclosure, in the step (3), a reagent system for the methylation is used, and the reagent system for the methylation further comprises a formic acid in addition to one or more of formaldehyde (e.g., an aqueous formaldehyde solution), paraformaldehyde, iodomethane, and dimethyl sulfate; preferably, the reagent system for the methylation used comprises paraformaldehyde and formic acid; more preferably, in the reagent system for the methylation, paraformaldehyde and formic acid are in a feeding mass ratio of 1:1-1:3, for example, 1:2.

According to an embodiment of the present disclosure, in the step (3), the reagent for the methylation and myosmine are in a feeding mass ratio of 1:1-1:10, for example, 1:2, 1:3, 1:4, 1:5, 1:6, 1:7, 1:8, or 1:9.

According to an embodiment of the present disclosure, in the step (3), a post-treatment is performed after the methylation reaction, which may include the following steps: adjusting a pH value with a basic reagent, performing extraction, and performing reduced pressure distillation to obtain a pure product; the basic reagent is selected from an aqueous sodium hydroxide solution; the pH value is greater than or equal to 8, and may be selected from 9, 10, 11, and 12. According to an embodiment of the present disclosure, in the step (3), a reaction solvent, myosmine, a ligand, and a metal catalyst are added to an autoclave at room temperature; a system is purged with nitrogen for three times, and then charged with hydrogen at a pressure of 1-1.5 MPa; the reaction is performed at 20-40 °C for 3-5 h; after the system is emptied and purged with nitrogen for 1-3 times, paraformaldehyde and formic acid are added for a reflux reaction for 3-6 h; after the reaction is completed, a post-treatment is performed to obtain a pure product.

According to an embodiment of the present disclosure, the extraction solvent in the reaction (e.g., used in the post-treatment in the step (1), step (2), and step (3)) may be selected from one or more of the following reagents: ethyl acetate, methyl *tert*-butyl ether, dichloromethane, and the like.

The present disclosure also provides a catalyst generated *in situ* from a ligand and a metal catalyst, wherein the metal catalyst is selected from Rh(COD)Cl₂, Ir(COD)Cl₂, Ru(COD)Cl₂, PdCl(PPh₃)₃, PdCl₂(PPh₃)₂, Ni(acac)₂, NiCl₂, and Ni(COD)₂; the ligand is selected from the following structures:

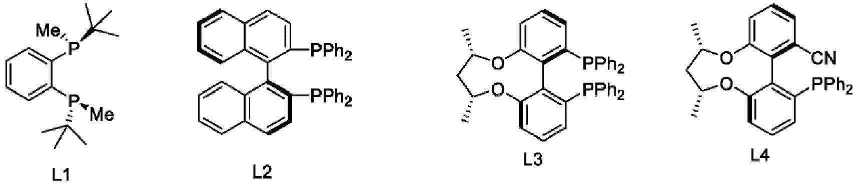

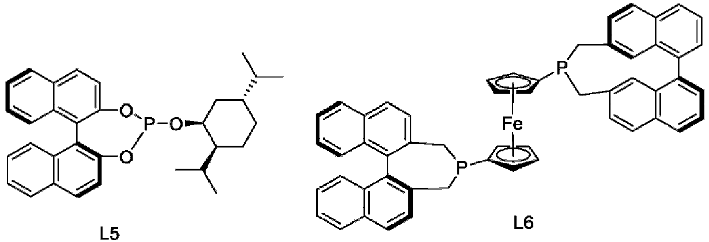

The present disclosure also provides use of the catalyst in a reduction reaction, wherein preferably, the catalyst is used in a carbonyl reduction reaction, and more preferably in an asymmetric carbonyl reduction reaction, for example, in the reaction of the step (3) described above.

### Beneficial Effects

1) By the synthesis process of the present disclosure, L-nicotine with an optical purity of more than 99.9% can be obtained, which is much higher than that of similar products in the current market, and the total yield of the synthesis reaches 50%-60%; the reaction materials are cheap and easy to obtain; the process is simple to operate, environment-friendly, and suitable for large-scale industrial production.
2) In the present disclosure, a specific ligand and metal catalyst are used to perform a reduction reaction, which can significantly improve the reaction quality.

### BRIEF DESCRIPTION OF THE DRAWINGS

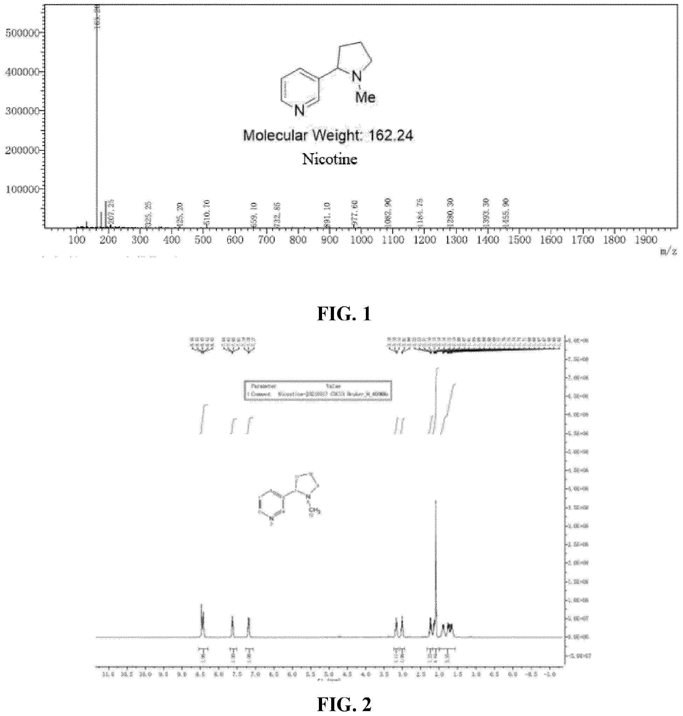

FIG. 1 is a mass spectrum of L-nicotine according to the present disclosure;
FIG. 2 is a nuclear magnetic resonance spectrum of L-nicotine according to the present disclosure;
FIG. 3 is an optical purity diagram of L-nicotine according to the present disclosure.

### DETAILED DESCRIPTION

The technical solutions of the present disclosure will be further illustrated in detail with reference to the following specific examples. It should be understood that the following examples are merely exemplary illustrations and explanations of the present disclosure, and should not be construed as limiting the protection scope of the present disclosure. All techniques implemented based on the content of the present disclosure described above are included within the protection scope of the present disclosure.

Unless otherwise stated, the starting materials and reagents used in the following examples are all commercially available products or can be prepared using known methods.

Optical detection instrument and method: detection instrument A (ultraviolet detector); a chiral chromatography column Daicel OD-H is used, the mobile phase is isopropanol/n-heptane = 5:95, the flow rate is 0.9 mL/min, the sample injection volume is 10 µL, the detection wavelength is 254 nm, and the column temperature is 25 °C.

### General reaction route:

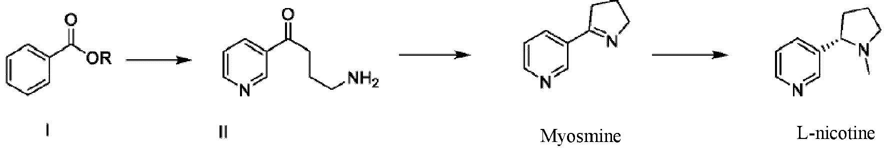

### Example 1: Preparation of intermediate compound of formula II

To a 50 L three-necked flask were added 20 kg of xylene, 5 kg of ethyl nicotinate (i.e., R = ethyl), 2.5 kg of potassium *tert*-butoxide, and 4 kg of *N*-vinylpyrrolidone at 30 °C. The system was heated to 100 °C for a reaction for 4 h, and then 3 kg of 15% hydrochloric acid was added dropwise over 3 h with the temperature controlled at 50 °C. After the dropwise addition, the system was heated to 90 °C for a reaction for 2 h, and then cooled to 30 °C. The system was adjusted to pH 7 with 10% sodium hydroxide, and then extracted with ethyl acetate (20 kg × 2). The organic phases were combined, dried, and concentrated under reduced pressure at 40 °C to obtain 4.11 kg of the compound of formula II with a liquid phase purity of 97.3% and a yield of 76.7%, which was used directly in the next step. ¹HNMR (CDCl₃, 400M) δ: 8.91 (d, J = 7.8 Hz, 1H), 8.51 (d, J = 8.0 Hz, 1H), 8.35 (d, J = 7.8 Hz, 1H), 7.68 (d, J = 8.0 Hz, 1H), 3.03 (t, J = 8.0 Hz, 2H), 2.81 (t, J = 8.0 Hz, 2H), 2.22-2.20 (m, 2H), 1.90-1.88 (br, 2H), LC-MS Calc: 164.21, Detec. M+1: 167.2.

### Example 2: Preparation of myosmine

To a 50 L three-necked flask were added 15 kg of tetrahydrofuran, 5 kg of water, and 5 kg of the compound of formula II at 20 °C. 1.5 kg of sodium hydroxide was added with the temperature of the system controlled at 40 °C. The reaction was performed at 40 °C until the compound of formula II was completely consumed. The system was extracted with dichloromethane (10 kg × 2). The organic phases were combined and concentrated under reduced pressure at 30 °C to obtain 4.35 kg of myosmine with a liquid phase purity of 97.1% and a yield of 97.8%, which was used directly in the next step. ¹HNMR (CDCl₃, 400M) δ: 8.58(d, J = 8.0 Hz, 1H), 8.45 (d, J = 7.8 Hz, 1H), 7.68 (d, J = 8.0 Hz, 1H), 7.24-7.20 (m, 1H), 4.13 (t, J = 4.0 Hz, 1H), 3.20-3.17 (m, 1H), 3.06-3.01 (m, 1H), 2.22-2.20 (m, 1H), 1.90-1.88 (m, 1H), 1.80-1.60 (m, 1H); LC-MS Calc: 146.19, Detec. M+1: 147.2.

### Example 3: Preparation of L-nicotine

To a 50 L autoclave were added 20 kg of tetrahydrofuran, 3 kg of myosmine, 1.5 g of ligand L2, and 1 g of Ir(COD)Cl₂ at 25 °C. The system was purged with nitrogen at a pressure of 0.3 MPa for three times, and then charged with hydrogen at a pressure of 1.2 MPa. The system was reacted at 30 °C for 4 h. After the system was emptied and purged with nitrogen at a pressure of 0.2 MPa for 2 times, 500 g of paraformaldehyde and 1 kg of formic acid were added for a reflux reaction at 95 °C for 5 h. The system was concentrated to remove the organic solvent, adjusted to pH 11 with an aqueous sodium hydroxide solution, and extracted with ethyl acetate (9 kg × 3). The organic phases were combined and concentrated to give a nicotine crude product, which was then distilled under reduced pressure to obtain a pure L-nicotine product as a colorless transparent liquid, with an optical purity of more than 99.9% ee. The pure product had a fraction weight of 2.46 and a yield of 73.9%. ¹HNMR (400 MHz, CDCl₃) δ: 8.47-8.44 (br, 2H), 7.61 (d, J = 8.0 Hz, 1H), 7.20-7.16 (m, 1H), 3.18 (t, J = 8.0 Hz, 1H), 3.11 (t, J = 8.0 Hz, 1H), 2.25-2.19 (m, 1H), 2.13-2.09 (m, 1H), 2.08 (s, 3H), 1.89-1.87 (m, 1H), 1.76-1.66 (m, 2H); LC-MS Calc: 162.24, Detec. M+1: 163.20.

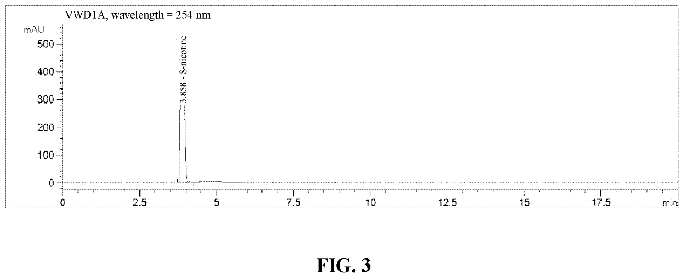

The resulting pure L-nicotine was subjected to optical detection. The results are shown in FIG. 3 and the table below:

| Serial number | Time | Peak area | Peak height | Peak width | Symmetry factor | Peak area% |
|---|---|---|---|---|---|---|
| 1 | 3.849 | 4435.1 | 546.6 | 0.1194 | 0.342 | 100.00 |

The embodiments of the present disclosure have been described above. However, the present disclosure is not limited to the embodiments described above. Any modification, equivalent, improvement, and the like made without departing from the spirit and principle of the present disclosure shall fall within the protection scope of the present disclosure.

## Claims

1. A preparation method for L-nicotine, having the following synthetic route: 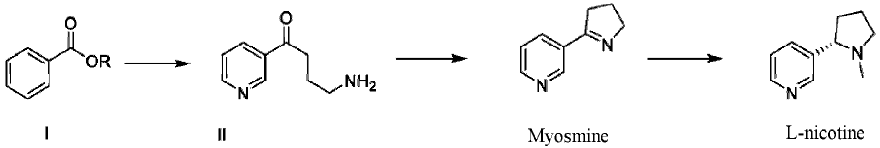 wherein the preparation method comprises the following steps:
(1) subjecting a compound of formula I to a substitution reaction with N-vinylpyrrolidone, and performing decarboxylation to obtain a compound of formula II;
(2) subjecting the compound of formula II to a cyclization reaction to obtain myosmine; and
(3) subjecting myosmine to reduction and methylation to obtain L-nicotine;
in the compound of formula I, R is selected from C₁-C₈ alkyl, phenyl, and benzyl.

2. The preparation method for L-nicotine according to claim 1, wherein in the step (1), the compound of formula I is subjected to the substitution reaction with N-vinylpyrrolidone under the action of a base; the base is selected from one or more of potassium hydroxide, sodium hydroxide, potassium *tert*-butoxide, sodium ethoxide, potassium carbonate, sodium hydride, butyl lithium, and methylmagnesium bromide.

3. The preparation method for L-nicotine according to claim 1 or 2, wherein in the substitution reaction of the step (1), one or more of the following reagents are used as a reaction solvent: toluene, xylene, tetrahydrofuran, ethanol, 2-methyltetrahydrofuran, and n-hexane.

4. The preparation method for L-nicotine according to any one of claims 1 to 3, wherein in the step (1), an acid is added for the decarboxylation reaction; the acid is selected from one or more of hydrochloric acid, phosphoric acid, sulfuric acid, formic acid, and acetic acid.

5. The preparation method for L-nicotine according to any one of claims 1 to 4, wherein in the step (2), the compound of formula II is subjected to the cyclization reaction under the action of a base; the base is selected from one or more of potassium hydroxide, sodium hydroxide, potassium *tert*-butoxide, sodium ethoxide, potassium carbonate, sodium hydride, triethylamine, butyl lithium, and methylmagnesium bromide; in the step (2), one or more of the following reagents are used as a reaction solvent: water, ethyl acetate, dichloromethane, *N,N-*dimethylformamide, tetrahydrofuran, ethanol, 2-methyltetrahydrofuran, *n*-hexane, and methyl *tert*-butyl ether.

6. The preparation method for L-nicotine according to claim 5, wherein in the step (2), the reaction solvent and the compound of formula II are in a feeding mass ratio of 20:1-2:1; the base and the compound of formula II are in a feeding mass ratio of 1:1-1:20.

7. The preparation method for L-nicotine according to any one of claims 1 to 6, wherein in the step (3), the reduction is performed in the presence of a ligand and a metal catalyst; in the step (3), the metal catalyst is selected from Rh(COD)Cl₂, Ir(COD)Cl₂, Ru(COD)Cl₂, PdCl(PPh₃)₃, PdCl₂(PPh₃)₂, Ni(acac)₂, NiCl₂, and Ni(COD)₂; in the step (3), the ligand is selected from the following structures:

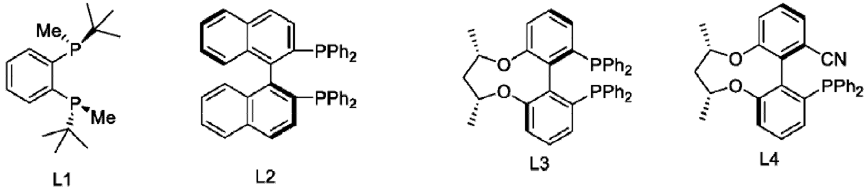

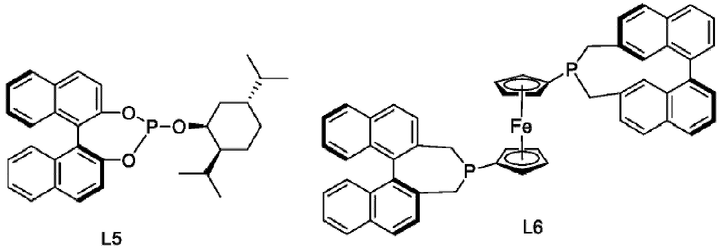

8. The preparation method for L-nicotine according to claim 7, wherein in the step (3), a reagent for the methylation is selected from one or more of formaldehyde (e.g., an aqueous formaldehyde solution), paraformaldehyde, iodomethane, and dimethyl sulfate; preferably, in the step (3), a reagent system for the methylation is used, and the reagent system for the methylation further comprises formic acid in addition to one or more of formaldehyde (e.g., an aqueous formaldehyde solution), paraformaldehyde, iodomethane, and dimethyl sulfate.

9. A catalyst generated *in situ* from a ligand and a metal catalyst, wherein the metal catalyst is selected from Rh(COD)Cl₂, Ir(COD)Cl₂, Ru(COD)Cl₂, PdCl(PPh₃)₃, PdCl₂(PPh₃)₂, Ni(acac)₂, NiCl₂, and Ni(COD)₂; the ligand is selected from the following structures:

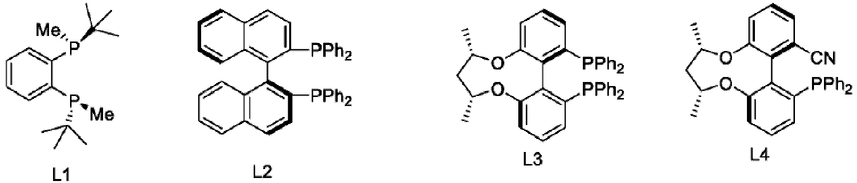

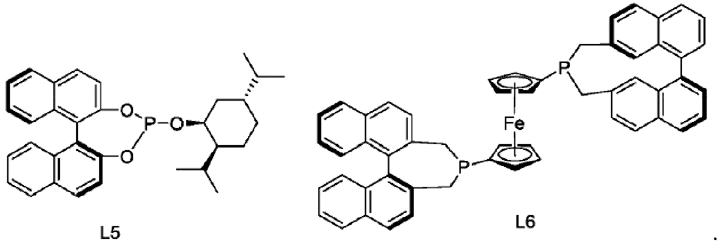

10. Use of the catalyst according to claim 9 in a reduction reaction, wherein preferably, the catalyst is used in a carbonyl reduction reaction.
